# EUROPEAN PATENT SPECIFICATION

(11) **EP 4 117 629 B1**
(45) Date of publication and mention of the grant of the patent: **15.04.2026**
(21) Application number: 21767112.2
(22) Date of filing: 26.02.2021
(51) Int. Cl.: A61K 9/00, A61K 9/06, A61K 31/407, A61P 25/36, A61K 31/485, A61K 47/02

(54) **COMPOSITIONS AND MEDICAL USE OF NALOXONE**
ZUSAMMENSETZUNGEN UND MEDIZINISCHE VERWENDUNG VON NALOXON
COMPOSITIONS ET UTILISATION MÉDICALE DE NALOXONE

(30) Priority: 11.03.2020 US 202062988161 P
(43) Date of publication of application: 18.01.2023
(73) Proprietor: Purdue Pharma L.P., Stamford, CT 06901 (US)
(72) Inventor: HUANG, Haiyong, Hugh, Princeton Junction, NJ 08550 (US); SHET, Manjunath, S., White Plains, NY 10607 (US)
(74) Representative: Maiwald GmbH
(86) International application number: PCT/US2021/019902
(87) International publication number: WO 2021/183303

(56) References cited:
- WO-A1-2019/231993
- WO-A1-2020/097279
- WO-A2-01/26642
- CA-A1- 2 990 387
- US-A- 4 500 515
- US-A1- 2014 357 657
- US-A1- 2017 368 029
- US-A1- 2018 169 006
- US-A1- 2019 054 080
- MCDONALD REBECCA ET AL: "Pharmacokinetics of concentrated naloxone nasal spray for opioid overdose reversal: Phase I healthy volunteer study : Concentrated naloxone nasal spray pharmacokinetics", vol. 113, no. 3, 16 November 2017 (2017-11-16), GB, pages 484 - 493, XP055856756, ISSN: 0965-2140, Retrieved from the Internet <URL:https://api.wiley.com/onlinelibrary/tdm/v1/articles/10.1111%2Fadd.14033> DOI: 10.1111/add.14033
- MCDONALD ET AL.: "Pharmacokinetics of concentrated naloxone nasal spray for opioid overdose reversal: Phase I healthy volunteer study", ADDICTION, vol. 113, 16 November 2017 (2017-11-16), pages 484 - 493, XP055856756

## Description

### FIELD OF THE INVENTION

The present invention relates to the field of pharmaceutical compositions for rescuing a subject from an opioid overdose and drug delivery systems thereof.

### BACKGROUND OF THE INVENTION

Pharmaceutical products are sometimes subject to abuse. For example, a particular dose of opioid analgesic may be more potent when administered parenterally as compared to the same dose administered orally. Abusing a pharmaceutical product may result in an overdose that could be fatal. Also, potent opioids can be used as toxic chemical agents intentionally or unintentionally to cause death in humans through exposing humans to lethal doses through aerosolizing or other means of dispersal.

Symptoms of opioid overdose include, but not limited to, loss of consciousness, unresponsiveness to outside stimulus, being awake but unable to talk, respiratory depression or respiratory cessation, vomiting, limp body, pale or clammy skin, bluish fingernails and lips, slow heartbeat, erratic heartbeat, no heartbeat and eventual death.

To counteract opioid overdose effects, emergency personnel or others may administer an antidote such as an intramuscular injection of an opioid antagonist. Given that the administered antidote needs to be absorbed into the bloodstream, there inevitably will be a lag from the time of antidote administration to the time that the antidote reaches therapeutic levels sufficient to effectively counteract the effects of the opioid. Unfortunately, this lag time can result in the antidote treatment not being effective enough in sufficient time to prevent morbidity and mortality due to the overdose. WO 2019/231993 A1, CA 2 990 387 A1, US 2017/368029 A1, WO 01/26642 A2, US 4 500 515 A and McDonald et al. Addiction, vol.113, no. 3 16 (2017) disclose compositions suitable for use in the treatment of opioid overdose.

There exists a need in the art for a pharmaceutical composition for rescuing a subject from opioid overdose, and a method of rescuing a subject from an opioid overdose which method provides a rapid onset of action.

### OBJECTS AND SUMMARY OF THE INVENTION

It is an object of the present invention to provide a parenteral pharmaceutical composition for rescuing a subject from an opioid overdose, or for preventing (or reducing the risk in) a subject from experiencing an opioid overdose.

In one embodiment, the parenteral formulation is suitable for intramuscular administration. In another embodiment, the parenteral formulation is suitable for subcutaneous administration.

It is an object of certain embodiments of the present invention to provide a medical use for rescuing a subject from an opioid overdose, or for preventing (or reducing the risk in) a subject from experiencing an opioid overdose.

It is an object of certain embodiments of the present invention to provide a medical use of prophylactically administering a pharmaceutical composition as disclosed herein to a subject (e.g., a first responder or a member of law enforcement) who is at risk of being exposed to a toxic amount of an opioid agonist (e.g., fentanyl, sufentanyl, carfentanyl, or a salt or derivative thereof).

It is an object of certain embodiments of the present invention to provide a drug delivery system for rescuing a subject from an opioid overdose, or from preventing (or reducing the risk in) a subject (e.g., a first responder or a member of law enforcement) from experiencing an opioid overdose.

Several investigators have reported that naltrexone is a useful adjunctive treatment following alcohol detoxification in alcohol-dependent human subjects (J. R. Volpicelli et al., Arch. Gen. Psychiatry, 49, 876, 1992; S. S. O'Malley et al. ibid., 49, 881, 1992). Similar results have been obtained in studies in rats and monkeys where naltrexone, naloxone, nalmefene, and other opioid antagonists have been shown to block the apparent rewarding effects of alcohol and reduce total alcohol consumption (D. R. Brown and S. G. Holtzman, Pharmacol. Biochem. Behav., 11, 567, 1979; L. D. Reid and G. A. Hunter, Alcohol, 1, 33, 1984; C. L. Hubbell et al., Alcohol, 3, 39, 1986; R. D. Myers et al., Alcohol, 3, 383, 1986; C. L. Hubbell et al., Alcohol, 8, 355, 1991; D. V. Gauvin et al., Alcohol, 10, 37, 1993). It has been suggested (N. P. Sykes, Palliative Medicine, 1996, 10, 135) that oral naloxone might have a therapeutic role in treatment of opioid induced constipation.

The compositions of the present invention may provide superior therapeutic effect because of the enhanced systemic absorption (e.g., quicker onset of action and superior pharmacokinetics) of the naloxone or a pharmaceutically acceptable salt thereof as compared to the same pharmaceutical composition without the adjuvant.

The above objects and others may be achieved by the present invention which is directed to a pharmaceutical composition for providing opioid overdose rescue to a subject, or for preventing an opioid overdose in a subject, wherein the pharmaceutical composition comprises naloxone or a pharmaceutically acceptable salt thereof, according to claim 1. The pharmaceutical composition comprises a therapeutically effective amount of naloxone or a pharmaceutically acceptable salt thereof, and a parenterally acceptable (absorption enhancing) amount of an adjuvant that is magnesium chloride that promotes, augments, or quickens the systemic absorption rate of the naloxone post intramuscular or subcutaneous injection. In certain non-limiting embodiments, the adjuvant may further comprise nitric oxide inducers, niacin, niacin derivatives, niacin metabolites phosphodiesterase inhibitors, angiotensin converting enzyme (ACE) inhibitors, angiotensin receptor blockers, calcium-channel blockers, nitrates or combinations thereof.

In certain embodiments, the pharmaceutical composition of the present invention comprises a therapeutically effective amount of naloxone or a pharmaceutically acceptable salt thereof and an (absorption-enhancing) effective amount of the pharmaceutically acceptable adjuvant wherein the composition provides a time to onset of action of the naloxone in 120 seconds or less post intramuscular or subcutaneous injection to a subject experiencing, or at risk of experiencing an opioid agonist overdose.

The composition provides a mean time to maximum plasma concentration of an naloxone in about 12 minutes or less post intramuscular or subcutaneous injection to a subject experiencing, or at risk of experiencing an opioid agonist overdose.

In certain embodiments, the composition provides a mean maximum plasma concentration of naloxone of at least about 0.5 ng/mL per 0.1 mg of naloxone or pharmaceutically acceptable salt thereof, post intramuscular or subcutaneous injection to a subject experiencing, or at risk of experiencing an opioid agonist overdose.

In certain embodiments, the composition provides an AUC_{0-inf} or AUC₀₋₂ of naloxone of at least about 0.6 (hr·ng/mL) per 0.1 mg of naloxone or a pharmaceutically acceptable salt thereof, post intramuscular or subcutaneous injection to a subject experiencing, or at risk of experiencing an opioid agonist overdose.

The present invention is directed to a medical use for an opioid overdose rescue or an opioid overdose prevention for a subject experiencing or at risk of experiencing an opioid agonist overdose, comprising intramuscularly, or subcutaneously administering to the subject a pharmaceutical composition as disclosed herein.

In certain embodiments, the present invention is directed to a drug delivery system comprising a device containing the pharmaceutical composition as disclosed herein. The device is suitable for delivering the pharmaceutical composition through injection. In certain embodiments, the composition is contained within a pre-filled syringe, a vial, an injection pen, or an auto-injector.

### BRIEF DESCRIPTION OF THE DRAWINGS

The above and other features of the present invention, their nature, and various advantages will become more apparent post consideration of the following detailed description, taken in conjunction with the accompanying drawings, in which:

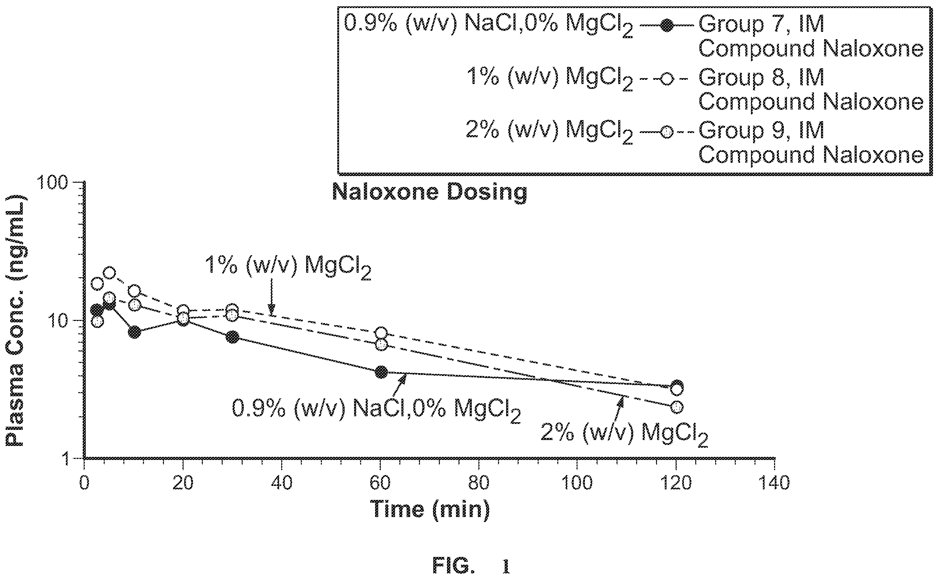

Figure 1 depicts the comparative mean plasma concentration profiles of a 0.6 mg dose of naloxone with different levels of MgCl₂ adjuvant (0%, 1% (w/v), and 2% (w/v)) after intramuscular administration in three dog subjects. For dog subjects receiving 0.6 mg dose of naloxone with 0% MgCl₂, 0.9% (w/v) NaCl was employed.

### DEFINITIONS

As used herein, the singular forms "a," "an," and "the" include plural references unless the context clearly indicates otherwise. Thus, for example, reference to "an opioid antagonist" includes a single opioid antagonist as well as a mixture of two or more different opioid antagonists; and reference to an "excipient" includes a single excipient as well as a mixture of two or more different excipients, and the like.

As used herein, the term "about" in connection with a measured quantity or time, refers to the normal variations in that measured quantity or time, as expected by one of ordinary skill in the art in making the measurement and exercising a level of care commensurate with the objective of measurement. In certain embodiments, the term "about" includes the recited number ±10%, such that "about 10" would include from 9 to 11, or "about 1 hour" would include from 54 minutes to 66 minutes.

As used herein, the term "active agent" refers to any material that is intended to produce a therapeutic, prophylactic, or other intended effect, whether or not approved by a government agency for that purpose. This term with respect to a specific agent includes the pharmaceutically active agent, and all pharmaceutically acceptable salts, solvates and crystalline forms thereof, where the salts, solvates and crystalline forms are pharmaceutically active.

As used herein, the terms "therapeutically effective" and an "effective amount" refer to that amount of an active agent or the rate at which it is administered needed to produce a desired therapeutic result.

As used herein, the term "absorption enhancing" refers to that amount of an adjuvant or the rate at which the adjuvant is administered needed to promote, augment, or quicken the systemic absorption of an active agent.

The term "subject" refers to a human or animal who has demonstrated a clinical manifestation of an opioid overdose suggesting the need for a rescue treatment, or who is at risk of being exposed to a toxic amount of an opioid. For example, in a first medical responder or law enforcement context, the subject is treated prophylactically with naloxone or pharmaceutically acceptable salt thereof. The term "subject" may include a person or animal (e.g., a canine) who is a patient being appropriately treated by a medical caregiver with an opioid to treat or prevent pain. The term "subject" may also include a person or animal who is inappropriately using an opioid through misuse, abuse, or through inadvertent exposure. The term "subject" may also include a first responder (such as an EMT responding to a case of opioid overdose), or a member of law enforcement, or a drug detecting canine, who are preparing to enter a locale where a toxic amount of an opioid or opioids may be found. The term "subject" may also include any person who appears to a non-clinically trained bystander to be experiencing one or more behaviors (such as unconsciousness, unresponsiveness, slowed breathing, or other behaviors suggestive of opioid-induced stupor or central nervous system depression) associated with excessive opioid exposure.

The terms "treatment of" and "treating" include the administration of an active agent(s) with the intent to lessen the severity of a condition.

The terms "prevention of" and "preventing" include the avoidance of the onset of a condition by a prophylactic administration of the active agent.

The term "condition" or "conditions" may refer to those medical conditions commonly recognized as the result of an opioid overdose, such as unresponsiveness, respiratory depression, vomiting, limp body, pale or clammy skin, bluish fingernails or lips, slow, erratic or no heartbeat, or a combination thereof, which can be treated, mitigated or prevented by a timely administration to a subject of an effective amount of an naloxone or pharmaceutically acceptable salt thereof. In certain embodiments, the term "condition" or "conditions" may refer to alcohol dependence or constipation.

The term "manic behavior" refers to a medical condition that may be characterized through physical and mental manifestations that may be expressed by one or more of the following symptoms: irritability, anxiety, aggressiveness, violence to self or others, hypersensitivity, hypervigilance, impulsivity, a compulsion to over-explain, sudden increase in energy levels, decreased need for sleep, hyperactivity, disorientation, incoherence, increase in risky behavior, inattentiveness, delusions, inflated self-esteem, grandiosity, distractibility, etc.

The term "combative behavior" refers to a subject's manifestation of violent, irritable, and/or aggressive symptoms that could result in physical or mental harm to the subject and/or to his surroundings and/or to a person administering a medical treatment, such as administration of an naloxone or pharmaceutically acceptable salt thereof.

The term "adjuvant" refers to an agent that is incorporated into a pharmaceutical composition to enhance the absorption of an active agent, e.g., by increasing Cₘₐₓ, shortening Tₘₐₓ, or increasing bioavailability, or a combination thereof. An adjuvant may be inactive in all other respects or may provide an intended or unintended pharmacological effect in addition to enhancing the absorption of an active agent.

A "toxic amount of an opioid agonist" may be understood by one skilled in the art (e.g., a clinician, a first responder, and the like) as the amount of opioid agonist which would most likely cause a serious adverse event (such as, respiratory failure, unresponsiveness, and slow breathing etc.). Such toxic amount may vary from one opioid agonist to another and from one individual subject to another.

The term "parenteral" means administration by injection, implantation or infiltration by a route such as intravenous, intrarterial, intracardiac, intraspinal, intraosseous, intrarticular, intrasynovial, intracutaneous, subcutaneous or intramuscular.

The term "injection" means administration to a discrete site through the skin or into tissue of a human or animal.

The term "implantation" means administration to a discrete site by embedding a pharmaceutical composition into the skin, tissue, muscles, tendons, joints, or other body parts of a human or animal

The term "infiltration" means administration into a discrete injection site, or surgical site or open wound.

T_{1/2} refers to the time for the plasma concentration of an active agent to decrease by half.

Tₘₐₓ refers to the time for the plasma concentration of an active agent to reach a Cₘₐₓ.

Cₘₐₓ refers to the maximum plasma concentration of an active agent.

AUC₀₋ₜ refers to the area under the curve of a plasma concentration as a function of time graph over a time period ranging from time 0 to time t.

AUC_{0-∞} refers to the area under the curve of a plasma concentration as a function of time graph over a time period ranging from time 0 extrapolated to infinite time.

The term "concurrently" as used herein means that a dose of one agent is administered prior to the end of the dosing interval of another agent. For example, a dose of naloxone or pharmaceutically acceptable salt thereof with a particular dosing interval would be concurrently administered with an anti-psychotic agent dose when administered within the dosing interval of the naloxone or pharmaceutically acceptable salt thereof.

The term "simultaneously" as used herein means that a dose of one agent is administered approximately at the same time as another agent, regardless of whether the agents are administered separately via the same or different routes of administration or in a single pharmaceutical composition or dosage form. For example, a dose of naloxone or a pharmaceutically acceptable salt thereof may be administered separately from, but at the same time as, a dose of an anti-psychotic agent.

The term "sequentially" as used herein means that a dose of one agent is administered first and thereafter a dose of another agent is administered second. For example, a dose of naloxone or a pharmaceutically acceptable salt thereof may be administered first, and thereafter a dose of an anti-psychotic agent may be administered second. The subsequent administration of the second agent may be inside or outside the dosing interval of the first agent.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to illuminate certain materials and methods and does not pose a limitation on scope.

### DETAILED DESCRIPTION

### Dosage Forms and Pharmaceutical Compositions

The invention is directed to a pharmaceutical composition comprising a therapeutically effective amount of an naloxone or a pharmaceutically acceptable salt thereof and a pharmaceutically acceptable adjuvant that promotes the absorption rate of the naloxone or a pharmaceutically acceptable salt thereof post intramuscular or subcutaneous injection, according to claim 1. The adjuvant is magnesium chloride and the adjuvant may further comprise nitric oxide inducers, niacin, niacin derivatives, niacin metabolites, phosphodiesterase inhibitors, angiotensin converting enzyme (ACE) inhibitors, angiotensin receptor blockers, calcium channel blockers, nitrates or combinations thereof. Such a pharmaceutical composition may provide for a quicker onset of action of the naloxone or a pharmaceutically acceptable salt thereof as compared to the same pharmaceutical composition without the adjuvant.

In certain embodiments, the invention is directed to the pharmaceutical composition comprising a therapeutically effective amount of naloxone or a pharmaceutically acceptable salt thereof and the parenterally acceptable adjuvant, wherein the formulation provides a time to onset of action (i.e., the first detectable therapeutic effect associated with administration of naloxone or pharmaceutically acceptable salt thereof, e.g., detectable lessening or reduction of any of the symptoms associated with opioid overdose) of about 120 seconds or less, about 90 seconds or less, about 60 seconds or less, or about 30 seconds or less post intramuscular or subcutaneous injection to a subject experiencing an opioid agonist overdose, or pretreating against potential opioid agonist exposure.

The invention is directed to the pharmaceutical composition comprising a therapeutically effective amount of naloxone or a pharmaceutically acceptable salt thereof and the parenterally acceptable adjuvant, wherein the formulation provides a mean time to maximum plasma concentration of the.naloxone of about 12 minutes or less, about 10 minutes or less, about 9 minutes or less, about 8 minutes or less, about 7 minutes or less, about 6 minutes or less, about 5 minutes or less, or about 4 minutes or less post intramuscular or subcutaneous injection to a population of subjects (e.g., healthy or otherwise healthy subjects).

In certain embodiments, the invention is directed to a pharmaceutical composition comprising a therapeutically effective amount of naloxone or pharmaceutically acceptable salt thereof and a parenterally acceptable adjuvant, wherein the formulation provides a mean time to maximum plasma concentration of the naloxone (Tₘₐₓ) that is shorter than the mean time to maximum plasma concentration of naloxone of a comparative formulation without an adjuvant, post intramuscular or subcutaneous injection to a population of subjects (e.g., healthy or otherwise healthy subjects). For instance, the mean Tₘₐₓ of the present invention may be, e.g., about 1.1 times shorter, about 1.2 times shorter, about 1.3 times shorter, about 1.4 times shorter, about 1.5 times shorter, about 1.6 times shorter, about 1.7 times shorter, about 1.8 times shorter, about 1.9 times shorter, about 2 times shorter, about 3 times shorter or about 4 times shorter than that of a comparative formulation without an adjuvant. In certain embodiments, Tₘₐₓ of the present invention may be, e.g., about 10% shorter, about 15% shorter, about 20% shorter, about 25% shorter, about 30% shorter, about 35% shorter, about 40% shorter, about 45% shorter, about 50% shorter, about 60% shorter, about 70% shorter, about 80% shorter, about 90% shorter, or any range therein, than that of a comparative formulation without an adjuvant.

In other embodiments, the invention is directed to a pharmaceutical composition comprising a therapeutically effective amount of naloxone or a pharmaceutically acceptable salt thereof and a parenterally acceptable adjuvant, wherein the formulation provides a mean maximum plasma concentration of naloxone at at least about 0.5 ng/mL per 0.1 mg naloxone or pharmaceutically acceptable salt thereof, post intramuscular or subcutaneous injection to a population of subjects (e.g., healthy or otherwise healthy subjects). In other embodiments, the formulation provides a mean maximum plasma concentration of naloxone at from about 0.5 ng/mL to about 25 ng/mL per 0.1 mg naloxone or pharmaceutically acceptable salt thereof, from about 0.5 ng/mL to about 10 ng/mL per 0.1 mg naloxone or pharmaceutically acceptable salt thereof, from about 1 ng/mL to about 5 ng/mL per 0.1 mg naloxone or pharmaceutically acceptable salt thereof, or from about 2.5 ng/mL to about 5 ng/mL per 0.1 mg naloxone or pharmaceutically acceptable salt thereof, post intramuscular or subcutaneous injection to a population of subjects (e.g., otherwise healthy subjects).

In certain embodiments, the invention is directed to a pharmaceutical composition comprising a therapeutically effective amount of naloxone or a pharmaceutically acceptable salt thereof and a parenterally acceptable adjuvant, wherein the formulation provides a mean maximum plasma concentration of naloxone (Cₘₐₓ) that is greater than the mean maximum plasma concentration of naloxone of a comparative formulation without an adjuvant, post intramuscular or subcutaneous injection to a population of subjects (e.g., healthy or otherwise healthy subjects). For instance, Cₘₐₓ may be about 1.1 times greater, about 1.2 times greater, about 1.3 times greater, about 1.4 times greater, about 1.5 times greater, about 1.6 times greater, about 1.7 times greater, about 1.8 times greater, about 1.9 times greater, or about 2 times greater, about 4 times greater, about 8 times greater, about 12 times greater or about 16 times greater than that of a comparative formulation without an adjuvant.

In other embodiments, the invention is directed to a pharmaceutical composition comprising a therapeutically effective amount of naloxone or a pharmaceutically acceptable salt thereof and a parenterally acceptable adjuvant, wherein the formulation provides a mean AUC_{0-inf} or AUC₀₋₂ of naloxone of at least about 0.6 (hr·ng/mL) per 0.1 mg naloxone or pharmaceutically acceptable salt thereof, post intramuscular or subcutaneous injection to a population of subjects (e.g., healthy or otherwise healthy subjects). In other embodiments, the formulation provides a mean AUC_{0-inf} or AUC₀₋₂ of naloxone of from about 0.6 (hr·ng/mL) to about 25 (hr·ng/mL) per 0.1 mg naloxone or pharmaceutically acceptable salt thereof, from about 0.6 (hr·ng/mL) to about 12 (hr·ng/mL) per 0.1 mg naloxone or pharmaceutically acceptable salt thereof, from about 1 (hr·ng/mL) to about 6 (hr·ng/mL) per 0.1 mg naloxone or pharmaceutically acceptable salt thereof, or from about 2.4 (hr·ng/mL) to about 4 (hr·ng/mL) per 0.1 mg naloxone or pharmaceutically acceptable salt thereof, post intramuscular or subcutaneous injection to a population of subjects (e.g., healthy otherwise healthy subjects).

In certain embodiments, the invention is directed to a pharmaceutical composition comprising a therapeutically effective amount of naloxone or a pharmaceutically acceptable salt thereof and a parenterally acceptable adjuvant, wherein the formulation provides AUC₀₋₂ that is greater than the AUC₀₋₂ of a comparative formulation without an adjuvant, post intramuscular or subcutaneous injection to a population of subjects (e.g., healthy or otherwise healthy subjects). For instance, AUC₀₋₂ may be about 1.1 times greater, about 1.2 times greater, about 1.3 times greater, about 1.4 times greater, about 1.5 times greater, about 1.6 times greater, about 1.7 times greater, about 1.8 times greater, about 1.9 times greater, or about 2 times greater, about 4 times greater, about 6 times greater, about 8 times greater or about 10 times greater than that of a comparative formulation without an adjuvant.

In certain embodiments, the pharmacokinetic values described herein may be obtained from an individual subject (healthy or in therapeutic need thereof) or from a plurality of subjects (healthy or in therapeutic need thereof) post a parenteral administration of any of the pharmaceutical compositions disclosed herein.

The role of the adjuvant is to promote or quicken the systemic absorption rate of the naloxone post intramuscular or subcutaneous injection. The adjuvant is magnesium chloride, being a vasodilator. The vasodilator may be an angiotensin converting enzyme (ACE) inhibitor, an angiotensin receptor blocker, a calcium channel blocker, a nitrate or magnesium chloride.

The adjuvant is magnesium chloride and is present in the pharmaceutical composition, at a concentration ranging from 0.5% (w/v) to 5% (w/v), preferably from 0.5% (w/v) to about 1% (w/v), from 0.5% (w/v) to about 1.5% (w/v), from about 1.5% (w/v) to about 2.5% (w/v), from 0.5% (w/v) to about 3.5% (w/v), from 0.5% (w/v) to about 3.0% (w/v), from about 2.5% (w/v) to about 3% (w/v), from about 2.0% (w/v) to about 4%(w/v), from about 2.0% (w/v) to about 3.0% (w/v), from about 4.5% (w/v) to 5% (w/v), about 0.9% (w/v), about 1% (w/v), about 2% (w/v), about 2.8% (w/v), about 3% (w/v), about 4.7% (w/v), 5% (w/v), of magnesium chloride in the pharmaceutical composition.

In certain embodiments, the adjuvant may further comprise a vasodilator that is an ACE inhibitor, e.g., enalapril, captopril, lisinopril, benazepril, enalaprilat, espirapril, fosinopril, moexipril, quinapril, ramipril, perindopril, trandolapril, pharmaceutically acceptable salts thereof or combinations thereof. In certain embodiments, the adjuvant may comprise a vasodilator that is an angiotensin receptor blocker, e.g., valsartan, losartan, irbesartan, telmisartan, eprosartan, candesartan, olmesartan, saprisartan, tasosartan, elisartan, pharmaceutically acceptable salts thereof or combinations thereof. In certain embodiments, the adjuvant may comprise a vasodilator that is a calcium channel blocker, e.g., amlodipine, anipamil, barnidipine, benidipine, bepridil, darodipine, diltiazem, efonidipine, felodipine, isradipine, lacidipine, lercanidipine, lidoflazine, manidipine, mepirodipine, nicardipine, nifedipine, niludipine, nilvadipine, nimodipine, nisoldipine, nitrendipine, perhexiline, tiapamil, verapamil, pharmaceutically acceptable salts thereof or combinations thereof. A person of skill in the art would readily recognize that the members of this list have the same or similar functions and thus would readily appreciate that each of these members can be used interchangeably alone or in combination.

In certain embodiments, the adjuvant further comprises a nitric oxide inducer. The nitric oxide inducer can be, e.g., an amino acid (e.g., arginine). The nitric oxide inducer can be, without limitation, L-arginine, L- homoarginine, N-hydroxy-L-arginine, nitrosated analogs thereof, nitrosylated analogs thereof, precursors thereof or combinations thereof. The nitrosated analogs may be, e.g., nitrosated L-arginine, nitrosated N-hydroxy-L- arginine, nitrosated L-homoarginine or combinations thereof. The nitrosylated analogs may be, e.g., nitrosylated L-arginine, nitrosylated N-hydroxy-L-arginine, nitrosylated L-homoarginine or combinations thereof. Also, the precursor may be, e.g., citrulline, ornithine, glutamine, lysine or combinations thereof. A person of skill in the art would readily recognize that the members of this list have the same or similar functions and thus would readily appreciate that each of these members can be used interchangeably alone or in combination. In one embodiment, the adjuvant further comprises L-arginine and is present in the pharmaceutical composition, e.g., at a concentration ranging from about 0.1% to about 50%, from about 5% to about 30%, from about 15% to about 25%, or about 20% (w/v) of L-arginine per pharmaceutical composition.

In certain embodiments, the nitric oxide inducer comprises arginase inhibitors, substrates for nitric oxide synthase, nitroglycerin, amyl nitrate, or combinations thereof. In certain embodiments, the arginase inhibitor comprises, e.g., N-hydroxy-L-arginine, 2(S)-amino-6-boronohexanoic or combinations thereof. In other embodiments, the substrate for nitric oxide synthase comprises cytokines, adenosine, bradykinin, calreticulin, bisacodyl, phenolphthalein, or combinations thereof. A person of skill in the art would readily recognize that the members of this list have the same or similar functions and thus would readily appreciate that each of these members can be used interchangeably alone or in combination.

In other embodiments, the adjuvant further comprises niacin, a niacin derivative, a niacin metabolite, or a combination thereof. The niacin derivative may be acifran, acipimox, niceritrol, isonicotinic acid, isonicotinohydrazide, pyridine carboxylic acid derivatives, 3-pyridine acetic acid, 5-methylnicotinic acid, pyridazine-4-carboxylic acid, pyrazine-2-carboxylic acid, or combinations thereof. In certain embodiments, the niacin derivative is an ester of nicotinic acid, e.g., an alkyl ester of nicotinic acid such as methyl nicotinate. In other embodiments, the niacin metabolite comprises nicotinuric acid, nicotinamide, 6-hydroxy nicotinamide, N-methylnicotinamide, nicotinamide-N-oxide, N-methyl-2-pyridone-5-carboxamide, N-methyl-4-pyridone-5-carboxamide, or combinations thereof. A person of skill in the art would readily recognize that the members of this list have the same or similar functions and thus would readily appreciate that each of these members can be used interchangeably alone or in combination. In certain embodiments, the adjuvant further comprises niacin and is present in the pharmaceutical composition at a concentration ranging from about 0.1% to about 15%, from about 0.5% to about 5%, about 1%, about 2%, or about 3% (w/v) of niacin per pharmaceutical composition.

In certain embodiments, the adjuvant further comprises a phosphodiesterase inhibitor. The phosphodiesterase inhibitor comprises phosphodiesterase 1 inhibitors, phosphodiesterase 2 inhibitors, phosphodiesterase 3 inhibitors, phosphodiesterase 4 inhibitors, phosphodiesterase 5 inhibitors, or combinations thereof. In other embodiments, the phosphodiesterase inhibitor comprises vinpocetine, EHNA (erythro-9-(2-hydroxy-3-nonyl)adenine), anagrelide, enoximine, cilomilast, etazolate, glaucine, ibudilast, mesembrine, rolipram, pentoxifylline, piclamilast, dipyridamole, acetildenafil, avanafil, sildenafil, tadalafil, udenafil, vardenafil, milrinone, amrinone or combinations thereof. A person of skill in the art would readily recognize that the members of this list have the same or similar functions and thus would readily appreciate that each of these members can be used interchangeably alone or in combination.

In certain embodiments, the pharmaceutical composition and dosage forms disclosed herein comprise from 0.5%, about 0.6%, about 0.7%, about 0.8%, about 0.9%, or about 1% to about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 35%, about 40%, about 45%, about 50%, about 60%, about 70%, or about 80% (w/v) of an adjuvant per dosage form. In certain embodiments, the pharmaceutical composition and dosage forms disclosed herein comprises to about 30%, from about 0.5% to about 25%, or from about 1% to about 20% (w/v) of an adjuvant per dosage form.

In certain embodiments, the pharmaceutical composition may further comprise a therapeutically effective amount of an antipsychotic agent to counteract manic behavior that may be triggered by the administration of the naloxone or pharmaceutically acceptable salt thereof and the sudden awakening of the subject from overdose to unfamiliar surroundings, possibly restrained in handcuffs or to a hospital bed, and possibly in the presence of rescue or law enforcement personnel (such as, first responders including ambulance operators, nurses, doctors, police officers, firefighters, Good Samaritans, etc.). Post intramuscular or subcutaneous administration of the pharmaceutical composition, a therapeutically effective amount of the antipsychotic agent is preferably bioavailable post opioid rescue or within a short time (e.g., about 12 minutes or less, about 10 minutes or less, about 8 minutes or less, about 5 minutes or less, about 3 minutes or less, or about 1 minute or less) after opioid overdose rescue. In this manner, when a subject awakens, e.g., after being rescued, the antipsychotic agent may inhibit or reduce any combative behavior that the subject would otherwise manifest post awakening. In some embodiments, the manic behavior comprises a physically combative behavior by the subject.

### Active Agents

The delivery systems and pharmaceutical compositions disclosed herein include an opioid antagonist being naloxone or its pharmaceutically acceptable salts. Pharmaceutically acceptable salts include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate, and the like; amino acid salts such as arginate, asparginate, glutamate and the like, and metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like.

In one embodiment, the opioid antagonist is naloxone hydrochloride.

In certain embodiments, the naloxone or a pharmaceutically acceptable salt thereof is present in a pharmaceutical formulation at about 0.05 mg/ml to about 10 mg/ml, about 0.1 mg/ml to about 5 mg/ml, about 0.3 mg/ml to about 4 mg/ml, about 1.0 mg/ml to about 4.0 mg/ml, and is adapted for parenteral administration.

In certain embodiments, the naloxone or a pharmaceutically acceptable salt thereof and the pharmaceutical formulation ( parenteral formulation) may comprise a dose (of naloxone base) from any of about 0.1 mg, about 0.2 mg, about 0.3 mg, about 0.4 mg, about 0.5 mg, or about 0.6 mg to any of about 0.7 mg, about 0.8 mg, about 0.9 mg, about 1.0 mg, about 2.0 mg, about 3.0 mg, about 4.0 mg, about 5.0 mg, about 10.0 mg, about 15.0 mg, or about 20.0 mg.

According to certain embodiments, the delivery systems and pharmaceutical compositions disclosed herein further comprise an anti-psychotic agent. In some embodiments, the anti-psychotic agent comprises butyrophenones, diphenylbutylpiperidines, phenothiazines, thioxanthenes, benzamides, tricyclics, benzisoxazoles or benzisothiazoles, phenylpiperazines, quinolinones, blonanserin, pimavanserin, sertindole, molindone, pharmaceutically acceptable salts thereof, or combinations thereof.

In some embodiments, the anti-psychotic agent is a butyrophenone. The butyrophenone may comprise benperidol, bromperidol, droperidol, haloperidol, melperone, pipamperone, timiperone, spiperone, pharmaceutically acceptable salts thereof, or combinations thereof.

In some embodiments, the anti-psychotic agent is a diphenylbutylpiperidine. The diphenylbutylpiperidine may comprise fluspirilene, penfluridol, pimozide, pharmaceutically acceptable salts thereof, or combinations thereof.

In some embodiments, the anti-psychotic agent is a phenothiazine. The phenothiazine may comprise acepromazine, chlorpromazine, cyamemazine, dixyrazine, fluphenazine, levomepromazine, mesoridazine, perazine, periciazine, perphenazine, pipotiazine, prochlorperazine, promazine, promethazine, prothipendyl, thioproperazine, thioridazine, trifluoperazine, triflupromazine, pharmaceutically acceptable salts thereof, or combinations thereof.

**In** some embodiments, the anti-psychotic agent is a thioxanthene. The thioxanthene may comprise chlorprothixene, clopenthixol, flupentixol, thiothixene, zuclopenthixol, pharmaceutically acceptable salts thereof, or combinations thereof.

**In** some embodiments, the anti-psychotic agent is a benzamide. The benzamide may comprise sulpiride, sultopride, veralipride, amisulpride, nemonapride, remoxipride, levosulpiride, tiapride, pharmaceutically acceptable salts thereof, or combinations thereof.

**In** some embodiments, the anti-psychotic agent may comprise a tricyclic compound. The tricyclic compound may comprise carpipramine, clocapramine, clorotepine, clotiapine, loxapine, mosapramine, asenapine, clozapine, olanzapine, quetiapine, zotepine, pharmaceutically acceptable salts thereof, or combinations thereof.

In some embodiments, the anti-psychotic agent is a benzisoxazole or benzisothiazole. The benzisoxazole or benzisothiazole may comprise iloperidone, lurasidone, paliperidone, paliperidone palmitate, perospirone, risperidone, ziprasidone, pharmaceutically acceptable salts thereof, or combinations thereof.

In some embodiments, the anti-psychotic agent is a phenylpiperazine or a quinolinone. The phenylpiperazine or quinolinone may comprise aripiprazole, aripiprazole lauroxil, brexpiprazole, cariprazine, pharmaceutically acceptable salts thereof, or combinations thereof.

In one embodiment, the anti-psychotic agent is haloperidol or a pharmaceutically acceptable salt thereof. In another embodiment, the opioid antagonist is naloxone or a pharmaceutically acceptable salt thereof and the anti-psychotic agent is haloperidol or a pharmaceutically acceptable salt thereof.

In certain embodiments, the anti-psychotic agent, per unit dose, comprises about 2 mg to about 40 mg, about 2 mg to about 20 mg, about 5 mg to about 15 mg, about 2 mg to about 10 mg, about 10 mg to about 20 mg, about 5 mg to about 10 mg, about 10 mg to about 15 mg, about 15 mg to about 20 mg, or about 7 mg to about 12 mg haloperidol or a pharmaceutically acceptable salt thereof suitable for intramuscular or subcutaneous administration.

In certain embodiments, the delivery systems and pharmaceutical compositions disclosed herein may further comprise an active agent selected from tranquilizers, CNS depressants, CNS stimulants, sedative hypnotics, or mixtures thereof.

In certain embodiments, the pharmaceutical composition and dosage forms disclosed herein may comprise from about 0.1%, about 0.2%, about 0.3%, about 0.4%, about 0.5%, about 0.6%, about 0.7%, about 0.8%, about 0.9%, about 1%, about 2%, about 3%, about 4%, about 5%, about 6%, or about 7% to about 8%, about 9%, about 10%, about 11%, about 12%, about 13%, about 14%, about 15%, about 16%, about 17%, about 18%, about 19%, about 20%, about 25%, about 30%, about 35%, about 40%, about 45%, about 50%, about 60%, about 70%, or about 80% (w/v) of naloxone or a pharmaceutically acceptable salt thereof, or a combination of the naloxone or a pharmaceutically acceptable salt thereof, adjuvant, and antipsychotic agent, per dosage form. In certain embodiments, the pharmaceutical composition and dosage forms disclosed herein may comprise from about 0.1% to about 80%, from about 0.5% to about 30%, or from about 1% to about 10% (w/v) of naloxone or a pharmaceutically acceptable salt thereof, or a combination of naloxone or a pharmaceutically acceptable salt thereof, adjuvant, and antipsychotic agent, per dosage form.

### Prophylactic Treatment

It is an object of certain embodiments of the present invention to provide a medical use to prevent or minimize an overdose of an opioid agonist in a subject that is at risk for exposure to an opioid agonist. For example, law enforcement personnel, first medical responders, or drug-sniffing canines can be pre-treated with an naloxone or a pharmaceutically acceptable salt thereof according to the present invention prior to entering an environment or locale (e.g., a crime scene or emergency situation) where they suspect that opioids (e.g., fentanyl, carfentanyl or sufentanyl) may have been intentionally or unintentionally released, or are otherwise present. Also, workers at environmental disaster areas involving opioids may be pretreated to avoid toxicity of opioids that may be present in the environment. In the embodiments directed to methods of prophylactic treatment, the administered compositions can include, but not be limited to the pharmaceutical compositions as disclosed herein. For example, the administration of naloxone or a pharmaceutically acceptable salt thereof for prophylactic treatment can utilize the presently disclosed formulations for intramuscular or subcutaneous administration.

### Pharmaceutically Acceptable Excipients

The pharmaceutical compositions according to the present invention may comprise one or more pharmaceutically acceptable carriers and excipients appropriate for intramuscular or subcutaneous administration. Examples of possible pharmaceutically acceptable carriers and excipients are described in the Handbook of Pharmaceutical Excipients, American Pharmaceutical Association (6^{th} Edition, 2009 Publication). Carriers and excipients suitable for intramuscular and subcutaneous formulations include, but are not limited to, antioxidants, buffering agents, diluents, surfactants, solubilizers, stabilizers, hydrophilic polymers, additional absorption or permeability enhancers, preservatives, osmotic agents, isotonicity agents, pH adjusting agents, solvents, co-solvents, viscosity agents, gelling agents, suspending agents or combinations thereof.

Suitable surfactants for the formulations disclosed herein include, but are not limited to Polysorbate 80 NF, polyoxyethylene 20 sorbitan monolaurate, polyoxyethylene (4) sorbitan monolaurate, polyoxyethylene 20 sorbitan monopalmitate, polyoxyethylene 20 sorbitan monostearate, polyoxyethylene (4) sorbitan monostearate, polyoxyethylene 20 sorbitan tristearate, polyoxyethylene (5) sorbitan monooleate, polyoxyethylene 20 sorbitan trioleate, polyoxyethylene 20 sorbitan monoisostearate, sorbitan monooleate, sorbitan monolaurate, sorbitan monopalmitate, sorbitan monostearate, sorbitan trilaurate, sorbitan trioleate, sorbitan tristearate, and the like, and combinations thereof. A person of skill in the art would readily recognize that the members of this list have the same or similar functions and thus would readily appreciate that each of these members can be used interchangeably alone or in combination.

Suitable isotonicity agents for the pharmaceutical compositions disclosed herein include, but are not limited to dextrose, lactose, sodium chloride, calcium chloride, magnesium chloride, sorbitol, sucrose, mannitol, trehalose, raffinose, various polyethylene glycol (PEG), hydroxyethyl starch, glycine, and the like, and combinations thereof. A person of skill in the art would readily recognize that the members of this list have the same or similar functions and thus would readily appreciate that each of these members can be used interchangeably alone or in combination.

Suitable suspending agents for the formulations disclosed herein include, but are not limited to microcrystalline cellulose, carboxymethylcellulose sodium NF, polyacrylic acid, magnesium aluminum silicate, xanthan gum, and the like, and mixtures thereof. A person of skill in the art would readily recognize that the members of this list have the same or similar functions and thus would readily appreciate that each of these members can be used interchangeably alone or in combination. In certain embodiments, the pharmaceutical compositions may include one or more suspending agents in an amount of from about 0.1 wt% to about 15 wt%, or from about 0.25 wt% to about 10 wt%, or from about 1 wt% to about 8 wt%, of the total weight of the pharmaceutical composition.

### Method of Providing Overdose Rescue

In certain embodiments, the present disclosure is directed to a medical use of providing opioid overdose rescue to a subject in need thereof. The method comprises administering to a subject in need thereof an effective amount of an naloxone or pharmaceutically acceptable salt thereof, the adjuvant, and optionally an antipsychotic agent, such that the onset of action of the naloxone is achieved in sufficient time to reverse or partially reverse the overdose. In certain embodiments, the present invention is intended to be urgently administered to a subject experiencing a medical emergency precipitated by opioid agonist overdose. In such circumstances, the pharmaceutical composition are typically administered by a medical practitioner, emergency medical technician, law enforcement member, family member, acquaintance, or bystander post observing the subject experiencing the symptoms of opioid agonist overdose. In some embodiments, the method may further comprise, before the administering step, identifying that the subject is experiencing an opioid agonist overdose.

The opioid agonist overdose treated by the present invention can result from any overdose resulting from any opioid or combination of opioids currently known or those that would be readily appreciated by an ordinary skilled artisan (in formulation and medical fields) for such use, including but not limited to any of the following: alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts thereof, and combinations thereof.

In certain embodiments, the naloxone or pharmaceutically acceptable salt thereof is administered to a subject in an effective amount to counteract the opioid agonist overdose. In certain embodiments, the optional anti-psychotic agent is co-administered to a subject with naloxone or pharmaceutically acceptable salt thereof and an adjuvant in an effective amount to prevent, reduce, or counteract a manic behavior. The manic behavior may be a physically or mentally combative behavior seen in some subjects immediately post recovery from the overdose.

In other embodiments, the naloxone or pharmaceutically acceptable salt thereof, adjuvant, and the optional anti-psychotic agent are all administered together as a combination in a single dosage form. In one embodiment, the naloxone or pharmaceutically acceptable salt thereof and adjuvant may be administered together as a combination and the optional anti-psychotic agent may be administered separately.

In certain embodiments, the optional anti-psychotic agent is administered to a subject before the subject returns to consciousness. In this manner, the subject may already experience a therapeutic effect of the anti-psychotic agent post awakening or shortly thereafter, which may serve to prevent the subject from engaging in a physically or mentally combative behavior after rescue from the opioid agonist overdose.

In some embodiments, the naloxone or pharmaceutically acceptable salt thereof, adjuvant, and the optional anti-psychotic agent are all administered via the same route of administration, i.e., intramuscular or subcutaneous. For example, the optional anti-psychotic agent may be administered via intravenous administration, nasal administration, sublingual or buccal administration, or by inhalation.

In one embodiment, the naloxone or pharmaceutically acceptable salt thereof, adjuvant, and the optional anti-psychotic agent are both administered to a subject in need thereof via intramuscular administration with a needle gauge ranging from about 18 gauge to about 27 gauge. For instance, the needle used for intramuscular administration of any of the pharmaceutical compositions described herein may be 18 gauge, 20 gauge, 21 gauge, 22 gauge, 23 gauge, 25 gauge, or 27 gauge.

In another embodiment, the naloxone or pharmaceutically acceptable salt thereof, adjuvant, and the optional anti-psychotic agent are administered to a subject in need thereof via subcutaneous administration with a needle gauge ranging from about 18 gauge to about 35 gauge. For instance, the needle used for subcutaneous administration of any of the pharmaceutical compositions described herein may be 18 gauge, 20 gauge, 21 gauge, 22 gauge, 23 gauge, 25 gauge, 27 gauge, 29 gauge, 31 gauge, or 33 gauge.

In some embodiments, the naloxone or pharmaceutically acceptable salt thereof, adjuvant, and the optional anti-psychotic agent are administered concurrently, simultaneously, or sequentially.

### Other Indications

Several investigators have reported that naltrexone is a useful adjunctive treatment following alcohol detoxification in alcohol-dependent human subjects (J. R. Volpicelli et al., Arch. Gen. Psychiatry, 49, 876, 1992; S. S. O'Malley et al. ibid., 49, 881, 1992). Similar results have been obtained in studies in rats and monkeys where naltrexone, naloxone, nalmefene, and other opioid antagonists have been shown to block the apparent rewarding effects of alcohol and reduce total alcohol consumption (D. R. Brown and S. G. Holtzman, Pharmacol. Biochem. Behav., 11, 567, 1979; L. D. Reid and G. A. Hunter, Alcohol, 1, 33, 1984; C. L. Hubbell et al., Alcohol, 3, 39, 1986; R. D. Myers et al., Alcohol, 3, 383, 1986; C. L. Hubbell et al., Alcohol, 8, 355, 1991; D. V. Gauvin et al., Alcohol, 10, 37, 1993). It has been suggested (N. P. Sykes, Palliative Medicine, 1996, 10, 135) that oral naloxone might have a therapeutic role in treatment of opioid induced constipation.

The compositions of the present invention may provide superior therapeutic effect because of the enhanced systemic absorption (e.g., quicker onset of action and superior pharmacokinetics) of the naloxone or a pharmaceutically acceptable salt thereof as compared to the same pharmaceutical composition without the adjuvant.

The amount of active agent in the pharmaceutical composition is effective to treat, counteract, or reduce the severity of the target indication, opioid overdose, and/or one or more of their symptoms.

### Drug Delivery Systems and Kits

In certain embodiments, the present invention is directed to a drug delivery system or to a kit containing an injection device and any of the pharmaceutical formulations disclosed herein. In certain embodiments, the injection device is pre-filled with the pharmaceutical formulation. In certain embodiments, the injection device comprises a syringe, a needle, a vial, an injection pen, or an autoinjector, which is pre-filled with the pharmaceutical formulation disclosed herein.

In certain embodiments, the drug delivery system or kit may comprise an active agent and an adjuvant in separate containers (e.g., separate vials, separate syringe barrels, separate compartments, and the like). In one embodiment, naloxone or a pharmaceutically acceptable salt thereof may be in one container and the adjuvant ( MgCl₂) may be in another container such that the naloxone or pharmaceutically acceptable salt thereof may be mixed prior to administration parenterally.

In certain embodiments, the naloxone or pharmaceutically acceptable salt thereof in the drug delivery system or kit may be in solution or in powder form. In certain embodiments, the adjuvant in the drug delivery system or kit may be in solution or in powder form.

In one embodiment, the drug delivery system or kit may comprise a solution of an naloxone or a pharmaceutically acceptable salt thereof in one container and the adjuvant ( MgCl₂) solution in another container. The naloxone solution and the adjuvant solution may be mixed prior to administration. In one embodiment, the naloxone solution may be in one compartment of an auto-injector and the adjuvant solution may be in another compartment in an auto-injector and the two solutions may be mixed in the auto-injector prior to administration parenterally. In another embodiment, the naloxone solution may be in one vial, the adjuvant solution may be in another vial, and the contents of the vials may be mixed prior to administration (e.g, by transferring the content of one vial into another vial with a syringe and needle which could be part of the kit described herein).

In one embodiment, the drug delivery system or kit described herein may comprise a solution of naloxone or a pharmaceutically acceptable salt thereof solution in one container and the adjuvant ( MgCl₂) powder in another container. The naloxone solution and the adjuvant powder may be mixed prior to administration. In one embodiment, the naloxone solution may be in one compartment of an auto-injector and the adjuvant powder may be in another compartment in an auto-injector and the powder and solution may be mixed in the auto-injector prior to administration. In another embodiment, the naloxone solution may be in one vial (or pre-filled syringe barrel or the like), the adjuvant powder may be in another vial, and the naloxone solution may be added to the adjuvant powder (e.g., by transferring the naloxone solution into the adjuvant powder container with a syringe and needle which could be part of the kit described herein) to suspend or dissolve the adjuvant powder prior to administration parenterally.

In one embodiment, the drug delivery system or kit described herein may comprise a powder of naloxone or a pharmaceutically acceptable salt thereof in one container and the adjuvant MgCl₂) solution in another container. The naloxone powder and the adjuvant solution may be mixed prior to administration parenterally. In one embodiment, the naloxone powder may be in one compartment of an auto-injector and the adjuvant solution may be in another compartment of an auto-injector and the powder and solution may be mixed in the auto-injector prior to administration. In another embodiment, the naloxone powder may be in one vial, the adjuvant solution may be in another vial (or pre-filled syringe barrel or the like), and the adjuvant solution may be added to the naloxone powder (e.g., by transferring the adjuvant solution into the naloxone powder container with a syringe and needle which could be part of the kit described herein) to suspend or dissolve the naloxone powder prior to administration parenterally.

In one embodiment, the drug delivery system or kit described herein may comprise a powder of naloxone or a pharmaceutically acceptable salt thereof in one container, the adjuvant ( MgCl₂) powder in another container, and a solvent in yet another container. The naloxone powder, the adjuvant powder, and a solvent may be mixed prior to administration. In one embodiment, the naloxone powder may be in one compartment of an auto-injector, the adjuvant powder may be in another compartment of an auto-injector, and a solvent may be in yet another compartment of an auto injection such that the powders may be suspended or dissolved in the solvent prior to administration parenterally. In another embodiment, the naloxone powder may be in one vial, the adjuvant powder may be in another vial, and the solvent may be in yet another vial (or pre-filled syringe barrel or the like), and the solvent may be added to the naloxone powder and/or to the adjuvant powder (e.g., by transferring the solvent into the naloxone powder and/or the adjuvant powder container(s) with a syringe and needle which could be part of the kit described herein) to suspend or dissolve the powders prior to administration parenterally.

In certain embodiments, the drug delivery system or kit described herein may comprise naloxone or a pharmaceutically acceptable salt thereof and the adjuvant ( MgCl₂) combined together in a powder form in one container, and a pharmaceutically acceptable solvent is stored in another container, prior to administration. In one embodiment, a powder of the naloxone and a powder of the adjuvant may be mixed together in one compartment of an auto-injector and a pharmaceutically acceptable solvent may be stored in another compartment in an auto injector such that the powder mixture may be suspended or dissolved in the solvent prior to administration. In another embodiment, a powder of the naloxone and a powder of the adjuvant may be mixed together in one vial, and the solvent may be in another vial (or pre-filled syringe barrel or the like), and the solvent may be added to the powder mixture (e.g., by transferring the solvent into the powder mixture container with a syringe and needle which could be part of the kit described herein) to suspend or dissolve the powder mixture prior to administration.

In certain embodiments, the drug delivery system or kit described herein may comprises a needle having a needle gauge ranging from about 18 gauge to about 35 gauge. For instance, the needle used for parenteral administration of any of the pharmaceutical compositions described herein (whether it is a separate needle or a needle of an autoinjector device) may be 18 gauge, 20 gauge, 21 gauge, 22 gauge, 23 gauge, 25 gauge, 27 gauge, 29 gauge, 31 gauge, or 33 gauge.

Concentration ranges and/or values of active agents and/or adjuvants expressed in % (w/v) disclosed previously refer to the final concentrations when all components are mixed together just prior to administration.

### Examples

The following examples are set forth to assist in understanding the invention and should not be construed as specifically limiting the invention described and claimed herein.

### Example 1: Naloxone Study Design

Absorption of Naloxone (0.6 mg base dose based on an equivalent amount of naloxone hydrochloride, 3 mg/mL concentration) with varying concentrations of MgCl₂ adjuvant (0% MgCl₂ in the saline samples, 1% MgCl₂ in water, and 2% MgCl₂ in water) was tested. Each Naloxone formulation was administered intramuscularly to three dog subjects. Eight blood samples at varying time points (pre-dose, 2.5 minutes post dose, 5 minutes post dose, 10 minutes post dose, 20 minutes post dose, 30 minutes post dose, 60 minutes post dose, and 120 minutes post dose) were taken from each subject to evaluate the pharmacokinetic profile of Naloxone absorption. Details of the Naloxone study design are summarized in Table 1 below with reference to Groups 1-3. The plasma concentration of Naloxone after intramuscular administration in dogs with varying concentration of MgCl₂ is summarized in Figure 1 and in Table 2 below.

**Table 1 - Naloxone Study Design**

| **Grp** | **Test Article** | **Dosing Routine** | **N=** | **Base Dose (mg)** | **Dosing Concentration (mg/mL)** | **Dose Volume (mL)** | **Vehicle** | **Blood Sampling Points** |
|---|---|---|---|---|---|---|---|---|
| 7 | Naloxone HCl | IM | 3 | 0.6 | 3 | 0.2 | 0.9% NaCl (saline) and 0% MgCl₂ | Pre-dose, 2.5 minutes, 5 minutes, 10 minutes, 20 minutes, 30 minutes, 60 minutes, and 120 minutes post dose |
| 8 | Naloxone HCl | IM | 3 | 0.6 | 3 | 0.2 | 1% MgCl₂ in water | |
| 9 | Naloxone HCl | IM | 3 | 0.6 | 3 | 0.2 | 2% MgCl₂ in water | |

**Table 2 - Naloxone Pharmacokinetic Data Summary**

| | **Group 7** | **Group 8** | **Group 9** |
|---|---|---|---|
| | **IM** | **IM** | **IM** |
| **Parameters** | **Naloxone** | **Naloxone** | **Naloxone** |
| | **0.9% NaCl and 0% MgCl₂** | **1% MgCl₂** | **2% MgCl₂** |
| Dose (mg) | 0.6 | 0.6 | 0.6 |
| Cₘₐₓ (ng/mL) | 14.0 | 26.3 | 17.6 |
| Tₘₐₓ (hr) | 0.111 | 0.0694 | 0.208 |
| T_{1/2} (hr) | 0.834 | 0.783 | 0.651 |
| MRTₗₐₛₜ (hr) | 0.753 | 0.696 | 0.693 |
| AUCₗₐₛₜ (hr·ng/mL) | 11.4 | 17.9 | 14.5 |
| AUC_{∞} (hr·ng/mL) | 13.4 | 21.5 | 15.7 |

As seen in Figure 1 and in Table 2, Naloxone formulations with MgCl₂ (groups 8 and 9) achieve a higher Cₘₐₓ than Naloxone formulations without MgCl₂ (group 7). It can also be observed from Figure 1 and Table 2 that Naloxone formulations with 1% MgCl₂ (group 8) shorten the Tₘₐₓ as compared to Naloxone formulations without MgCl₂ (group 7). Additionally, the calculated AUCₗₐₛₜ observed in Naloxone formulations with MgCl₂ (groups 8 and 9) is greater than that of Naloxone formulations without MgCl₂ (group 7). Without being construed as limiting, it is observed that based on the above data and Figure 1, MgCl₂ enhances absorption of Naloxone by increasing the Cₘₐₓ and/or decreasing the Tₘₐₓ and/or increasing the AUC₀₋₂.

In the foregoing description, numerous specific details are set forth, such as specific materials, dimensions, processes parameters, etc., to provide a thorough understanding of the present invention. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is simply intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment.

The present invention has been described with reference to specific exemplary embodiments thereof. The scope of the invention is defined by the appended claims.

## Claims

1. A parenteral formulation comprising a therapeutically effective amount of naloxone or a pharmaceutically acceptable salt thereof and a parenterally acceptable adjuvant, wherein the adjuvant is magnesium chloride and is present in the parenteral formulation from 0.5% w/v to 5% w/v and wherein the formulation provides a mean time to maximum plasma concentration of naloxone of 12 minutes or less upon intramuscular or subcutaneous administration.

2. The parenteral formulation of claim 1, wherein the formulation provides a time to onset of opioid antagonistic action of 120 seconds or less, 90 seconds or less, 60 seconds or less, or 30 seconds or less, upon intramuscular or subcutaneous injection to a subject experiencing an opioid agonist overdose.

3. The parenteral formulation of any of claims 1-2, wherein the adjuvant promotes the systemic absorption rate of the naloxone or pharmaceutically acceptable salt thereof upon injection as compared to the same formulation without the adjuvant.

4. The parenteral formulation of any one of claims 1-3, wherein the naloxone or pharmaceutically acceptable salt thereof comprises naloxone hydrochloride.

5. The parenteral formulation of any of claims 1-4 for use in providing opioid overdose rescue to a subject, wherein the parenteral formulation is intramuscularly or subcutaneously administered to a subject in need thereof.

6. A drug delivery system comprising an injection device containing a parenteral formulation of any of claims 1-4.

7. A kit comprising a parenteral formulation of any of claims 1-4 and at least one container, wherein the naloxone or pharmaceutically acceptable salt thereof is stored in one container and the adjuvant is stored in another container, or the naloxone or pharmaceutically acceptable salt thereof and the adjuvant are stored together in one container, and wherein the naloxone or pharmaceutically acceptable salt thereof and the adjuvant are independently in a solution form or in a powder form.

8. The kit of claim 7, wherein the naloxone or pharmaceutically acceptable salt thereof comprises naloxone hydrochloride.

9. The kit of any one of claims 7-8, further comprising an injection device.

10. The kit of claim 9, wherein the injection device is an auto-injector, wherein preferably the auto-injector is pre-filled with a solution of the naloxone or pharmaceutically acceptable salt thereof, a solution of the parenterally acceptable adjuvant, or both.

11. The kit of any one of claims 7-10, further comprising a syringe and a needle.

## Patentansprüche

1. Parenterale Formulierung umfassend eine therapeutisch wirksame Menge an Naloxon oder einem pharmazeutisch annehmbaren Salz davon und ein parenteral annehmbares Adjuvans, wobei das Adjuvans Magnesiumchlorid ist und in der parenteralen Formulierung in einer Menge von 0,5 % w/v bis 5 % w/v vorhanden ist, und wobei die Formulierung eine mittlere Zeit bis zur maximalen Plasmakonzentration von Naloxon von 12 Minuten oder weniger nach intramuskulärer oder subkutaner Verabreichung bereitstellt.

2. Parenterale Formulierung nach Anspruch 1, wobei die Formulierung eine Zeit bis zum Einsetzen der Opioid-antagonistischen Wirkung von 120 Sekunden oder weniger, 90 Sekunden oder weniger, 60 Sekunden oder weniger oder 30 Sekunden oder weniger nach intramuskulärer oder subkutaner Injektion bei einem Patienten mit einer Opioid-Agonisten-Überdosierung bereitstellt.

3. Parenterale Formulierung nach einem der Ansprüche 1-2, wobei das Adjuvans die systemische Absorptionsrate des Naloxons oder pharmazeutisch annehmbaren Salzes davon nach der Injektion im Vergleich zu derselben Formulierung ohne das Adjuvans fördert.

4. Parenterale Formulierung nach einem der Ansprüche 1-3, wobei das Naloxon oder pharmazeutisch annehmbare Salz davon Naloxonhydrochlorid umfasst.

5. Parenterale Formulierung nach einem der Ansprüche 1-4 zur Verwendung bei der Rettung eines Patienten mit einer Opioid-Überdosierung, wobei die parenterale Formulierung einem Patienten, der dies benötigt, intramuskulär oder subkutan verabreicht wird.

6. Arzneimittelabgabesystem umfassend eine Injektionsvorrichtung enthaltend eine parenterale Formulierung nach einem der Ansprüche 1-4.

7. Kit umfassend eine parenterale Formulierung nach einem der Ansprüche 1-4 und mindestens einen Behälter, wobei das Naloxon oder pharmazeutisch annehmbare Salz davon in einem Behälter und das Adjuvans in einem anderen Behälter gelagert wird oder das Naloxon oder pharmazeutisch annehmbare Salz davon und das Adjuvans zusammen in einem Behälter gelagert werden, und wobei das Naloxon oder pharmazeutisch annehmbare Salz davon und das Adjuvans unabhängig voneinander in Lösung oder in Pulverform vorliegen.

8. Kit nach Anspruch 7, wobei das Naloxon oder pharmazeutisch annehmbare Salz davon Naloxonhydrochlorid umfasst.

9. Kit nach einem der Ansprüche 7-8, ferner umfassend eine Injektionsvorrichtung.

10. Kit nach Anspruch 9, wobei die Injektionsvorrichtung ein Autoinjektor ist, wobei der Autoinjektor vorzugsweise mit einer Lösung des Naloxons oder pharmazeutisch annehmbaren Salzes davon, einer Lösung des parenteral annehmbaren Adjuvans oder beidem vorgefüllt ist.

11. Kit nach einem der Ansprüche 7-10, ferner umfassend eine Spritze und eine Nadel.

## Revendications

1. Formulation parentérale comprenant une quantité thérapeutiquement efficace de naloxone ou d'un sel pharmaceutiquement acceptable de celle-ci et d'un adjuvant acceptable par voie parentérale, dans laquelle l'adjuvant est du chlorure de magnésium et est présent dans la formulation parentérale à raison de 0,5 % p/v à 5 % p/v et dans laquelle la formulation permet d'atteindre une concentration plasmatique maximale de naloxone en 12 minutes ou moins en moyenne après administration intramusculaire ou sous-cutanée.

2. Formulation parentérale selon la revendication 1, dans laquelle la formulation permet un délai d'action antagoniste des opioïdes de 120 secondes ou moins, 90 secondes ou moins, 60 secondes ou moins, ou 30 secondes ou moins, après injection intramusculaire ou sous-cutanée à un sujet présentant un surdosage d'agonistes opioïdes.

3. Formulation parentérale selon l'une quelconque des revendications 1-2, dans laquelle l'adjuvant favorise le taux d'absorption systémique de la naloxone ou d'un sel pharmaceutiquement acceptable de celle-ci lors de l'injection par rapport à la même formulation sans l'adjuvant.

4. Formulation parentérale selon l'une quelconque des revendications 1-3, dans laquelle la naloxone ou un sel pharmaceutiquement acceptable de celle-ci comprend du chlorhydrate de naloxone.

5. Formulation parentérale selon l'une quelconque des revendications 1-4 pour utilisation dans le traitement d'une surdose d'opioïdes chez un sujet, dans laquelle la formulation parentérale est administrée par voie intramusculaire ou sous-cutanée à un sujet qui en a besoin.

6. Système d'administration de médicament comprenant un dispositif d'injection contenant une formulation parentérale selon l'une quelconque des revendications 1-4.

7. Kit comprenant une formulation parentérale selon l'une quelconque des revendications 1-4 et au moins un récipient, dans lequel la naloxone ou un sel pharmaceutiquement acceptable de celle-ci est stocké dans un récipient et l'adjuvant est stocké dans un autre récipient, ou le naloxone ou un sel pharmaceutiquement acceptable de celle-ci et l'adjuvant sont stockés ensemble dans un seul récipient, et dans lequel la naloxone ou un sel pharmaceutiquement acceptable de celle-ci et l'adjuvant se présentent indépendamment sous forme de solution ou sous forme de poudre.

8. Kit selon la revendication 7, dans lequel la naloxone ou un sel pharmaceutiquement acceptable de celle-ci comprend du chlorhydrate de naloxone.

9. Kit selon l'une quelconque des revendications 7-8, comprenant en outre un dispositif d'injection.

10. Kit selon la revendication 9, dans lequel le dispositif d'injection est un auto-injecteur, dans lequel, de préférence, l'auto-injecteur est prérempli avec une solution de naloxone ou d'un sel pharmaceutiquement acceptable de celle-ci, une solution d'adjuvant acceptable par voie parentérale, ou les deux.

11. Kit selon l'une quelconque des revendications 7-10, comprenant en outre une seringue et une aiguille.
